# EUROPEAN PATENT SPECIFICATION

(11) **EP 2 010 101 B1**
(45) Date of publication and mention of the grant of the patent: **06.01.2010**
(21) Application number: 06744599.9
(22) Date of filing: 28.04.2006
(51) Int. Cl.: A61F 2/24

(54) **HEART VALVE PROSTHESIS**
HERZKLAPPENPROTHESE
PROTHÈSE DE VALVE CARDIAQUE

(30) Priority: 04.04.2006 RU 2006110832
(43) Date of publication of application: 07.01.2009
(73) Proprietor: Samkov, Alexandr V., Moscow 117465 (RU)
(72) Inventor: Samkov, Alexandr V., Moscow 117465 (RU)
(74) Representative: Andrae, Steffen
(86) International application number: PCT/IB2006/001066
(87) International publication number: WO 2007/113609

(56) References cited:
- EP-A1- 0 220 097
- EP-A1- 1 155 666
- DE-A1- 3 409 005
- GB-A- 2 281 371
- US-A- 5 522 886
- US-A1- 2005 049 697

## Description

### BACKGROUND OF THE INVENTION

One type of artificial heart valves is the mechanical heart valve, generally manufactured from artificial materials such as titanium, titanium alloys, graphite and others.

In a common design, mechanical heart valves include two leaflets held in a casing by hinges. When open, the blood flow through a bi-leaflet valve forms a central stream, between the two leaflets and two side streams. Deficiencies associated with bi-leaflet designs include turbulence, velocity gradients, non-synchronous opening of the leaflets and recirculation of the blood resulting in an increased burden on the heart.

Tri-leaflets designs also have been developed. Known in the art, for instance, is a heart valve having flat leaflets as disclosed in U.S. Patent No. 5,207,707, issued to Gourley on May 4, 1993. Flat leaflets also are used in the heart valve disclosed in Russian Patent document RU 2,173,969, published in 2001.

U.S. Patent No. 6,569,197 B2, issued to Samkov et al. on My 27, 2003, discloses an artificial heart valve prosthesis having an angular body which includes two flanges of different thickness and which houses three leaflets, each having a flat and a concave-spherical surface.

A tri-leaflet mechanical heart valve which includes three fan-shaped convexo-concave leaflets is disclosed in U.S. Patent Application Publication No. 2002/0022879 A1, published on February 21, 2002, and U.S. Patent No. 6,896,700, issued on May 24, 2005, both to Lu et al.

Both bi-leaflet and tri-leaflet mechanical heart valves often are associated with thrombosis and thromboembolism. Some tri-leaflet heart valve designs promote formation of stagnant zones in the blood flow, thus increasing the danger of blood clotting. In other cases, tri-leaflet valves continue to generate blood flow turbulence. Designs that place a high mechanical load on the hinge mechanisms result in an increased likelihood of early failure of the implant. Difficult machining is still another disadvantage associated with some of the existing tri-leaflet heart valves.

A need continues to exist therefore for improving mechanical heart valve prostheses. More specifically, a need continues to exist for tri-leaflet artificial heart valves that reduce or eliminate problems associated with existing designs.

### SUMMARY OF THE INVENTION

The invention generally relates to artificial heart valve prostheses, more specifically to a tri-leaflet artificial heart valve.

In one example, a heart valve prosthesis includes three leaflets attached to an annulus, each leaflet having convexo-concave surfaces and a non-uniform thickness.

In one embodiment, a heart valve prosthesis, includes a closing element having three flaps and an annulus for housing the closing element, the annulus having an interior surface. At a top region, the interior surface includes two sets of ledges. Ledges within each set are evenly spaced about the interior surface, a ledge in one set alternating with a ledge in the other set.

In a further embodiment, a heart valve prosthesis includes a closing element having three flaps, an annulus for housing the closing element, the annulus having an annulus axis and an interior surface and three ledges formed at the interior surface by three intersecting cylindrical surfaces evenly disposed within the annulus, each cylindrical surface having a cylindrical axis displaced from the annulus axis. At each ledge there are two sockets for attaching the flaps to the interior surface.

It is also described a leaflet shaped for a tri-leaflet artificial heart valve prosthesis. The leaflet has convexo-concave surfaces and a non-uniform thickness.

The invention has many advantages. For instance, the tri-leaflet artificial heart valve prosthesis disclosed herein resembles the human aortic valve and is expected to provide long and reliable service. The invention decreases or minimizes blood flow turbulence and reduces or eliminates formation of stagnant zones. It provides for a controlled reverse flow, thereby reducing or preventing blood clotting. In preferred embodiments, six cylindrical surfaces are used to shape the upper region of the annulus. Together with the three leaflets, the cylindrical surfaces give rise to essentially independent blood flow channels for washing or flushing the hinges that connect the leaves to the annulus, thus reducing the likelihood of thromboembolism. Other aspects of the invention promote good opening and closing conditions and minimize blood recirculation. Furthermore, the invention can reduce hydraulic resistance and improve the utilization of the valve cross sectional area. In specific aspects the invention also relates to an artificial heart valve prosthesis having an easy to manufacture casing.

### BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:

Fig. 1 is a view on a diametrical plane of a heart valve prosthesis with flaps in the closed position.

Fig. 2 is a view of convex and concave surfaces of a flap of the invention, with each surface being a fragment of a torus.

Fig. 3 is a top view of a heart valve prosthesis with flaps in the closed position.

Fig. 4 is a top view of a heart valve prosthesis with flaps in the open position.

Fig. 5 is a bottom view of a heart valve prosthesis with flaps in the open position.

Fig. 6 is a view on a diametrical plane of the heart valve prosthesis with flaps in the open position.

### DETAILED DESCRIPTION OF THE PREFERRED EMBODIMENTS

The above and other features of the invention including various novel details of construction and combinations of parts, and other advantages, will now be more particularly described with reference to the accompanying drawings and pointed out in the claims. It will be understood that the particular method and device embodying the invention are shown by way of illustration and not as a limitation of the invention. The principles and features of this invention may be employed in various and numerous embodiments without departing from the scope of the invention as claimed.

The invention generally relates to artificial heart valve prostheses. More specifically, the invention relates to tri-leaflet mechanical heart valves.

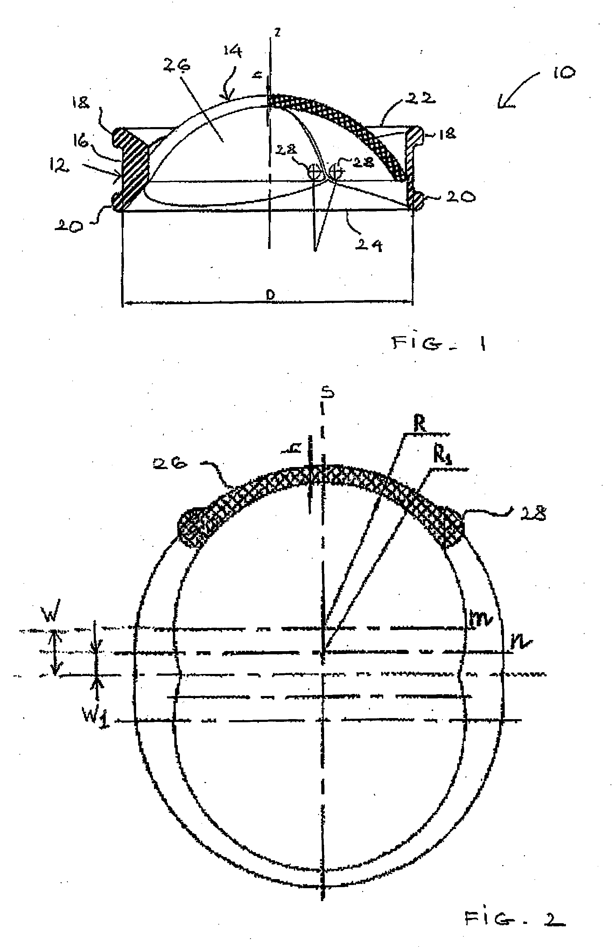

Shown in FIG. 1 is heart valve prosthesis 10, which includes annulus 12, housing closing element 14. Annulus 12 has a central axis, annulus axis Z, and an outer surface 16 with an outer diameter referred to herein as annulus diameter D. Preferably, at least a portion of surface 16 is cylindrically shaped.

Flanges 18 and 20 are positioned, respectively, at top region 22 and bottom region 24 of annulus 12. The top region is that in which the closing element opens. Preferably, flanges 18 and 20 are the same.

Annulus 12 is fabricated from a suitable material, often titanium or titanium-based. In one example, the annulus, also referred to as "casing", is doped with carbon ions.

For example, the annulus can be manufactured using an ion implantation apparatus which includes a vacuum chamber designed for processing multiple work pieces, e.g., casings. The vacuum chamber can include a plurality of rotatable holders, mounted on a rotatable disk in an arrangement in which only one holder is on a diametrical direction.

The ion implantation process can include propagating an ion beam along an ion beam pathway, rotating a disk having a plurality of holders to bring a casing supported by a holder in the ion beam pathway and rotating the holder to expose different surfaces of the casing to the ion beam. Preferably, not more than one holder is on a diametrical direction.

During the ion implantation process, the annulus can be moved by a method which includes rotating a disk around a disk rotational axis to impart a first movement component to the annulus being supported by a holder mounted on the disk; and spinning the holder around a holder rotational axis to impart a second movement component to the annulus. The annulus is tilted with respect to the disk rotational axis, to the holder rotational axis and with respect to the pathway of the ion beam.

Closing element 14 includes three flaps 26, shown in FIG. 1 in the closed position. Each of the flaps, also referred to as "leaflets", is attached to annulus 12 through two hinge mechanisms 28. Hinge mechanisms 28 can use, for example, protrusions, ridges, ledges, lugs, pivots, bearings or other hinge devices known in the art. Hemispherical pivots are preferred.

In one example, flaps 26 are attached to annulus 12 by an arrangement such as described in U.S. Patent No. 6,569,197 B2, issued to Samkov et al. on May 27, 2003.
For instance, recesses can be provided to engage bearings, with the recesses preferably having a lateral cylindrical surface and a concave, e.g., spherical bottom.

As disclosed in U.S. Patent No. 6,569,197 B2, recesses also can be provided in the form of a triad of blind holes being in communication via lateral surfaces. The triad preferably can have a central hole larger than the two side holes. The central hole is intended for mounting a flap bearing while the two side holes allow for blood evacuation from the hinge area. In one example, the smaller holes have a lateral cylindrical surface with a smooth concave bottom. As a variant, they can be semispherical.

Flaps 26 can be fabricated from a titanium or graphite substrate, coated with pyrolytic carbon, also known as pyrocarbon, or can be made entirely from pyrocarbon. Other suitable materials also can be employed. In a specific example, flaps 26 are manufactured as disclosed in WO /2006/117630, filed on April 29, 2005 and in PCT Patent Application filed concurrently herewith under Attorney Docket No. 105.5WO and entitled " Method for Manufacturing a Leaflet for Heart Valve Prostheses".

For instance, a carbon-containing precursor, present in a gas having a flow direction, is pyrolyzed to produce a pyrocarbon deposit on a substrate. The substrate has a fixed orientation with respect to the flow direction. A leaflet is formed from the deposit wherein a conjugate axis of the leaflet is oriented along the flow direction. In many leaflet designs the conjugate axis refers to the conjugate axis of the lugs. The leaflet can be formed before, during or after separation of the substrate from the deposit. In preferred examples the substrate is made of graphite.

Preferably, flaps 26 are identical and each has convexo-concave surfaces, a symmetry plane S and a flap thickness h, as shown in FIG. 2. In a preferred embodiment, h is not uniform but varies from symmetry plane S to a region at or near hinge mechanism 28. Further preferred are flaps in which the value of h increases from symmetry plane S towards the regions at or near hinge mechanisms 28. In specific examples h is increased from symmetry plane S to hinge mechanisms 28 by a factor within the range of from about 1.13 to about 1.35.

According to the invention, each of convex and concave surfaces of flap 26 is a segment, also referred to as "fragment" or "part" of a toroidal surface. In geometry, a torus is a shape generated by revolving a circle about an axis coplanar with the circle. One of the most common examples of a torus has the shape of a doughnut.

In the embodiment shown in FIG. 2, the concave surface of flap 26 is generated by revolving a circle of radius R about axis m, while the convex surface is generated by revolving a circle of radius R₁ about axis n. R and R₁ preferably are in the range of from about 0.3 to about 0.6 of annulus diameter D. The distance w, between axis m and the center of the circle having radius R, is in the range within from about O.lofR to about 0.3 of R. The distance w₁, between axis n and the center of the circle having radius R₁, is in the range within from about 0.1 of R₁ to about 0.3 of R₁. Preferably, the magnitude of R is different form that of R₁.

In other examples of the invention, each of the leaflets is convexo-concave and has a uniform thickness h'. Its surface is shaped as a fragment or part of a torus generated by revolving a circle of radius R' about an axis. R' can have a magnitude in the range of from about 0.6 and about 0.8 of annulus diameter D and the axis preferably is located at a distance w' in the range of from about 0.4 to about 0.6 of R' with respect to the center of the circle having radius R'.

Specific examples of how the flaps are attached to and engage with the interior of the heart valve casing are further described below.

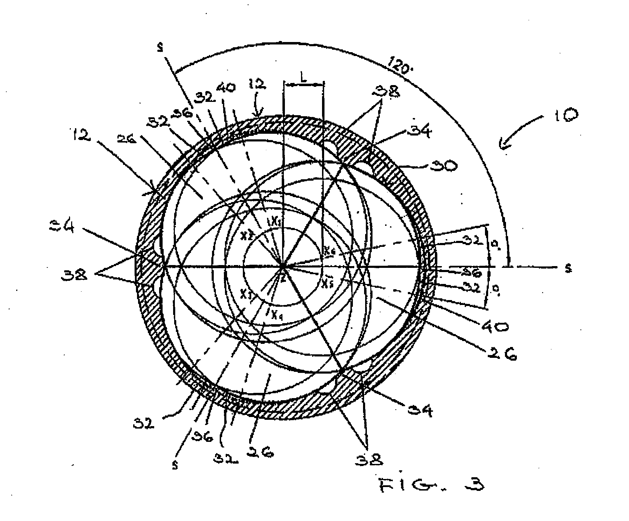

FIG. 3 is a top view of heart valve prosthesis 10 according to the invention which includes annulus 12 and flaps 26 in the closed position. Annulus 12 has interior surface 30. At top region 22, interior surface 30 of annulus 12 is shaped by three pairs of intersecting cylindrical surfaces 32 disposed at 120 degrees (°) of one another and preferably having the same diameter. The diameter of cylindrical surfaces 32 can be in the range of from about 0.5 to about 0.7 of annulus diameter D. Cylindrical axes x (x₁ - x₆) of cylindrical surfaces 32 are displaced from annulus axis Z by a distance L. Preferably L is in the range of from about 1/8 to about 1/4 of annulus diameter D.

Within each pair, cylindrical surfaces 32 have axes xₐ and x_{b}, e.g., x₁ and x₂, which are displaced from one another. If an arc of a circle is described with respect to annulus axis Z, the displacement of cylindrical axis xₐ with respect to cylindrical axis x_{b} is in the range of from about 18° to about 40°.

Intersections of cylindrical surfaces 32 described above generate two types of protrusions or ridges at the top region of interior surface 30, specifically three ledges 34 and three ledges 36. Ledges 34 preferably are evenly disposed within annulus 12, as are ledges 36. More specifically, the centers of ledges 36 are disposed at 120° from one to the next, as are the centers of ledges 34. As shown in FIG. 3, each ledge 34 alternates with a ledge 36. Thus each ledge 34 is positioned between two ledges 36.

In preferred embodiments, ledges 34 are different from ledges 36. According to the invention, ledges 34 are larger than ledges 36.

Two sockets 38 for engaging hinge mechanisms 28 of flaps 26 are recessed within surface 30 at each ledge 34. Preferably sockets 38 are hemispherically shaped to receive hemispherical pivots.

Ledges 3 6 receive peripheral edges of flaps 26 in the closed position.

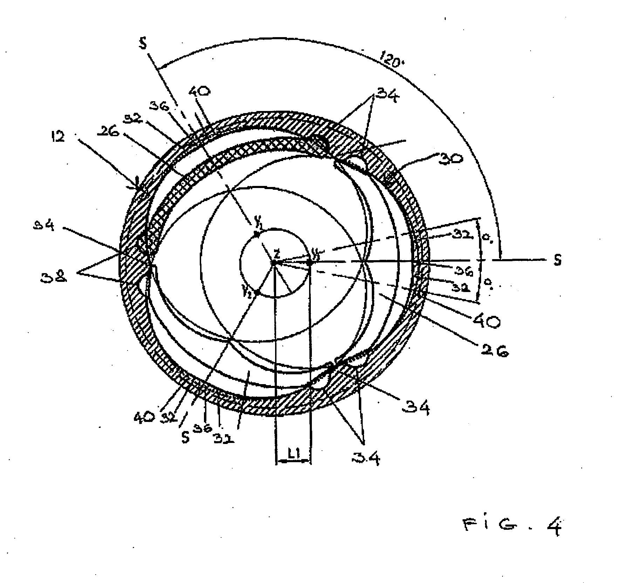

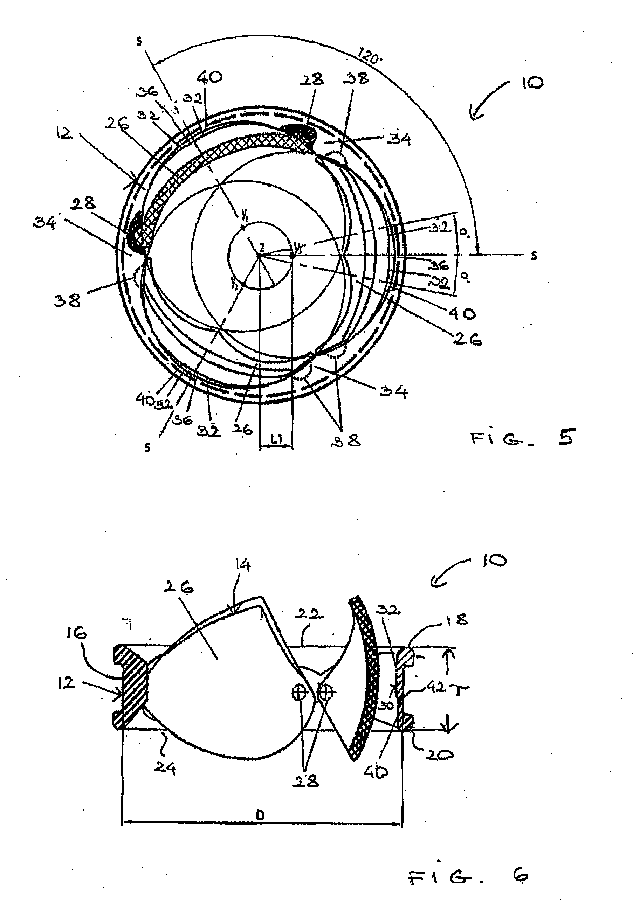

At bottom region 24, interior surface 30 of annulus 12 is shaped by three cylindrical surfaces 40 preferably having the same diameter and disposed at 120° with respect to one another, as shown in FIG. 4, which is a top view of heart valve prosthesis 10 with flaps 26 in the open position and in FIG. 5, which is a bottom view of heart valve prosthesis 10 also in the open position. Cylindrical surfaces 40 can have a diameter within the range of from about 0.6 to about 0.8 of annulus diameter D. Cylindrical axes y, specifically, y₁ - y₃, of cylindrical surfaces 40 are displaced by a distance L1 from annulus axis Z. Preferably, L1 is in the range of from about 1/10 to about 1/5 of annulus diameter D.

The segments most recessed into interior surface 30, formed by cylindrical surfaces 40 at bottom region 24, are opposite, preferably diametrically opposite, to ledges 34 at top region 22. Ledges 36 at top region 22 preferably are essentially lined up with the most recessed segments formed by cylindrical surfaces 40 at bottom region 24.

One example of a transition in the shape of interior surface 30 from top region 22 to bottom region 24 is shown in FIG. 6. Shown in FIG. 6 is heart valve prosthesis 10, including annulus 12 and flaps 26, shown in the open position. Within annulus height T, interior surface 30 includes an upper portion shaped by pairs of cylindrical surfaces 32, a lower portion shaped by cylindrical surfaces 40 and a transition region 42. Transition region 42 can be step-wise or tapered and preferably is located within the lower half with respect to height T. Other placements can be employed for transition region 42.

In the manufacturing process, the dimensions of heart valve prosthesis 10 are sized for its intended use. For example, annulus diameter D can be in the range of from about 17 millimeters (mm) to about 23 mm. Annulus height T can be in the range of from about 0.2 D to about 0.4 D. The approximate wall thickness of annulus 12 can be in the range of from about 3 mm to about 5 mm; at recessed regions, annulus 12 can be as thin as from about 2.5 mm to about 3.5 mm.

During operation, flaps 26 act as occluders for the opening and closing of the valve, which can operate from 0 to about 200 times a minute. In one cycle, flaps 26, initially in a closed position, open by turning about an imaginary axis that connects opposite hinge mechanisms 28, before their installation and in an equilibrium position. The blood flow thus is direct. At the end of the direct flow, flaps 26 turn back to their closed position.
Specifically, in the closed position, peripheral edges of flaps 26 engage or make contact with ledges 36. Ledges 34 and 36 together with flaps 26 provide independent flow channels for washing the region of hinge mechanisms 28.

In further examples, the heart valve prosthesis has an annulus with an interior surface that is shaped by the intersection of three cylindrical surfaces evenly disposed with respect to the interior circumference of the annulus and having the same diameter. The cylindrical axes of the three surfaces are shifted with respect to annulus axis Z by a length, L', preferably within the range of from about 1/6 to about 1/4 of the diameter D. Three evenly spaced ledges are formed at the intersection of the cylindrical surfaces. Each ledge is provided with recesses for engaging leaflet hinge mechanisms. For hemisherically shaped pivots, the recesses preferably are hemispherically shaped sockets.

The artificial heart valve of the invention can further include means for attachment at the implantation site, such as, for instance, a sewing ring made from woven materials. Other arrangements for attachment also can be used.

While this invention has been particularly shown and described with references to preferred embodiments thereof, it will be understood by those skilled in the art that various changes in form and details may be made therein without departing from the scope of the invention encompassed by the appended claims.

## Claims

1. A heart valve prosthesis (10), comprising:
a. an annulus (12) with two flanges (18,20) for housing the closing element (14), the annulus having an interior surface (30);
b. a closing element (14) of three convexo-concave leaflets (26), each having two hinge mechanisms (28) for engaging with the annulus; **characterised in that**,
c. a top region (22) of the interior surface (30) is shaped by three pairs of intersecting cylindrical surfaces (32) disposed at 120° of one another, said cylindrical surfaces (32) having a diameter within the range from 0.5 to 0.7 of annulus diameter D and cylindrical axes x₁ - x₆ of said cylindrical surfaces are displaced from annulus axis Z by a distance L, which is in the range of from 1/8 to 1/4 of annulus diameter D, wherein within each pair, said cylindrical surfaces (32) have axes, which are displaced from one another in the range of from 18° to 40° if an arc of a circle is described with respect to annulus axis Z;
d. a bottom region (24) of the interior surface (30) is shaped by three pairs of intersecting cylindrical surfaces (40) disposed at 120° of one another; said cylindrical surfaces (40) having a diameter within the range of from 0.6 to 0.8 of annulus diameter D and cylindrical axes y₁ - y₃, of said cylindrical surfaces (40) are displaced by a distance L1 from annulus axis Z, which L1 is in the range of from 1/10 to 1/5 of annulus diameter D.
e. a set of three larger ledges (34) and a set of three lesser ledges (36) formed at the interior surface (30) of the annulus by three pairs of intersecting cylindrical surfaces evenly disposed within the annulus, each larger ledge has two sockets (38) for engaging hinge mechanisms (28) in the three leaflets (26) and lesser ledges (36) engage peripheral edges of the three flaps (26) when in the closed position.

2. The heart valve prosthesis of Claim 1, wherein each convexo-concave leaflet (26) has a non-uniform thickness increasing 1,13 1,35 times from a leaflet symmetry plane (5) to a hinge mechanism (28), concave surface of each leaflet being a part of a torus surface, which torus is generated by revolving a circle of radius R about axis m, while the convex surface of each leaflet being a part of a torus surface, which torus is generated by revolving a circle of radius R₁ about axis n, the distance W, between axis m and the center of the circle having radius R, is in the range within from 0.1 of R to 0.3 of R₁ the distance W₁, between axis n and the center of the circle having radius R₁, is in the range within from 0.1 of R₁ to 0.3 of R₁, both R and R₁ are in the range of from 0.3 to 0.6 of annulus diameter D and the magnitude of R is less than of R₁.

## Patentansprüche

1. Herzklappenprothese (10), die aufweist:
a. einen Ring (12) mit zwei Flanschen (18, 20) zur Aufnahme des Verschlusselements (14), wobei der Ring eine Innenoberfläche (30) aufweist;
b. ein Verschlusselement (14) aus drei konvex-konkaven Blättern (26), die jeweils zwei Gelenkmechanismen (28) zur Wechselwirkung mit dem Ring aufweisen, **dadurch gekennzeichnet, dass**
c. ein oberer Abschnitt (22) der Innenoberfläche (30) von drei Paaren sich überschneidender zylindrischer Oberflächen (32) geformt wird, die unter 120° gegeneinander angeordnet sind, wobei die zylindrischen Oberflächen (32) einen Durchmesser aufweisen, der innerhalb des Bereichs von 0,5 bis 0,7 des Ringdurchmessers D liegt, und Zylinderachsen x₁ - x₆ der genannten zylindrischen Oberflächen gegenüber der Achse Z des Rings um einen Abstand L versetzt sind, der im Bereich von 1/8 bis 1/4 des Ringdurchmessers D beträgt, wobei in jedem Paar die zylindrischen Oberflächen (32) Achsen aufweisen, die gegeneinander im Bereich von 18° bis 40° versetzt sind, wenn ein Kreisbogen bezüglich der Ringachse Z geschlagen wird;
d. ein Bodenabschnitt (24) der Innenoberfläche (30) von drei Paaren sich überschneidender zylindrischer Oberflächen (40) geformt wird, die 120° gegeneinander versetzt sind; wobei die zylindrischen Oberflächen (40) einen Durchmesser im Bereich von 0,6 bis 0,8 des Ringdurchmessers D aufweisen und die Zylinderachsen y₁ - y₃ der Zylinderoberflächen (40) um einen Abstand L1 gegen die Ringachse Z versetzt sind, wobei L1 im Bereich von 1/10 bis 1/5 des Ringdurchmessers D liegt;
e. ein Satz von drei größeren Vorsprüngen (34) und ein Satz von drei geringeren Vorsprüngen (36), die an der Innenoberfläche (30) des Rings von drei Paaren von sich überschneidenden zylindrischen Oberflächen gebildet werden, die gleichmäßig innerhalb des Rings angeordnet sind, wobei jeder größere Vorsprung zwei Hülsen (38) zur Wechselwirkung mit den Gelenkmechanismen (28) an den drei Blättern (26) aufweist und die geringeren Vorsprünge (36) mit den Umfangskanten der drei Klappen (26) wechselwirken, wenn sich diese in der geschlossenen Stellung befinden.

2. Herzklappenprothese nach Anspruch 1, wobei jedes konvexkonkave Blatt (26) eine nicht gleichförmige Dicke aufweist, die von einer Symmetrieebene (S) des Blattes zu ₁einem Gelenkmechanismus (28) um das 1,13- bis 1,35-fache zunimmt, wobei die konkave Oberfläche eines jeden Blattes Teil einer Torusoberfläche ist, wobei der Torus erzeugt wird durch Rotieren eines Kreises mit dem Radius R um die Achse m, wohingegen die konvexe Oberfläche eines jeden Blattes Teil einer Torusoberfläche ist, wobei der Torus erzeugt wird durch Rotieren eines Kreises mit dem Radius R₁ um die Achse n, wobei der Abstand W zwischen der Achse m und dem Mittelpunkt des Kreises mit dem Radius R im Bereich von 0,1 von R bis 0,3 von R₁ beträgt und der Abstand W₁ zwischen der Achse n und dem Mittelpunkt des Kreises mit dem Radius R₁ im Bereich von 0,1 von R₁ bis 0,3 von R₁ liegt, wobei sowohl R als auch R₁ im Bereich von 0,3 bis 0,6 des Ringdurchmessers D liegen und der Wert von R geringer ist als der von R₁.

## Revendications

1. Prothèse de valve cardiaque (10) comprenant :
a. un anneau (12) doté de deux brides (18, 20) pour abriter l'élément de fermeture (14), l'anneau ayant une surface intérieure (30) ;
b. un élément de fermeture (14) à trois feuillets convexo-concaves (26), ayant chacune deux mécanismes d'articulation (28) pour entrer en prise avec l'anneau ;
**caractérisée en ce que**
c. une zone supérieure (22) de la surface intérieure (30) est façonnée par trois paires de surfaces cylindriques sécantes (32) disposées à 120° l'une par rapport à l'autre, lesdites surfaces cylindriques (32) ayant un diamètre compris entre 0,5 et 0,7 de diamètre de l'anneau D et les axes cylindriques x₁-x₆ desdites surfaces cylindriques étant déplacés de l'axe de l'anneau Z sur une distance L, qui est comprise entre 1/8 et 1/4 du diamètre de l'anneau D, où dans chaque paire, lesdites surfaces cylindriques (32) ont des axes, qui sont déplacés l'un par rapport à l'autre de 18° à 40°, si un arc de cercle est décrit relativement à l'axe de l'anneau Z ;
d. une zone inférieure (24) de la surface intérieure (30) est façonnée par trois paires de surfaces cylindriques sécantes (40), disposées à 120° l'une par rapport à l'autre ; lesdites surfaces cylindriques (40) ayant un diamètre compris entre 0,6 et 0,8 du diamètre de l'anneau D et les axes cylindriques y₁-y₃ desdites surfaces cylindriques (40) étant déplacés sur une distance L1 par rapport à l'axe de l'anneau Z, ladite L1 étant comprise entre 1/10 et 1/5 du diamètre d'anneau D.
e. un kit de trois rebords plus grands (34) et un kit de trois rebords plus petits (36) formés sur la surface intérieure (30) de l'anneau par trois paires de surfaces cylindriques sécantes disposées de manière uniforme dans l'anneau, chaque rebord plus grand ayant deux supports (38) pour mettre en prise les mécanismes d'articulation (28) dans les trois feuillets (26) et les rebords plus petits (36) mettant en prise les bords périphériques des trois feuillets (26) quand ils sont dans la position fermée.

2. Prothèse de valve cardiaque selon la revendication 1, dans laquelle chaque feuillet convexo-concave (26) a une épaisseur non-uniforme augmentant 1,13-1,35 fois par rapport à un plan de symétrie du feuillet (S) sur un mécanisme d'articulation (28), la surface concave de chaque feuillet faisant partie d'une surface de torus, ledit torus étant généré par la rotation d'un cercle de rayon R autour d'un axe m, tandis que la surface convexe de chaque feuillet fait partie d'une surface de torus, ledit torus étant généré par la rotation d'un cercle de rayon R₁ autour d'un axe n, la distance W, entre l'axe m et le centre du cercle ayant un rayon R, étant comprise entre 0,1 de R et 0,3 de R₁, et la distance W₁, entre l'axe n et le centre du cercle ayant un rayon R₁, étant comprise entre 0,1 de R₁ et 0,3 de R₁, R et R₁ étant compris entre 0,3 et 0,6 du diamètre de l'anneau D et l'amplitude de R étant inférieure à celle de R₁.
